# EUROPEAN PATENT SPECIFICATION

(11) **EP 1 756 141 B1**
(45) Date of publication and mention of the grant of the patent: **09.12.2009**
(21) Application number: 04817655.6
(22) Date of filing: 10.10.2004
(51) Int. Cl.: C07K 1/00, C07K 7/06, C07K 1/06

(54) **PROCESS FOR THE PREPARATION OF PEPTIDES**
VERFAHREN ZUR HERSTELLUNG VON PEPTIDEN
PROCESSUS DE PREPARATION DE PEPTIDES

(30) Priority: 14.06.2004 IN MU06482004
(43) Date of publication of application: 28.02.2007
(73) Proprietor: USV Ltd., Govandi, Mumbai 4000 88 (IN)
(72) Inventor: Saksena, Divya Lal, Govandi, Mumbai 400 088 (IN); Mishra, Shrikant, Govandi, Mumbai 400 088 (IN); Muralidharan, Chandrakesan, Govandi, Mumbai 400 088 (IN); Thakare, Milind, Govandi, Mumbai 400 088 (IN); Patil, Nilesh, Govandi, Mumbai 400 088 (IN); Khare, Aruna, Govandi, Mumbai 400 088 (IN)
(74) Representative: Schön, Christoph
(86) International application number: PCT/IN2004/000315
(87) International publication number: WO 2005/121164

(56) References cited:
- WO-A-03/093302
- WO-A-2004/092202
- US-A- 5 318 899
- SCARBOROUGH R M ET AL: "DESIGN OF POTENT AND SPECIFIC INTEGRIN ANTAGONISTS. PEPTIDE ANTAGONISTS WITH HIGH SPECIFICITY FOR GLYCOPROTEIN IIB-IIIA" JOURNAL OF BIOLOGICAL CHEMISTRY, AMERICAN SOCIETY OF BIOLOGICAL CHEMISTS, BALTIMORE, MD, US, vol. 268, no. 2, 15 January 1993 (1993-01-15), pages 1066-1073, XP000336707 ISSN: 0021-9258
- DELAET N G J ET AL: "Convenient solid phase synthesis method for the preparation of cysteine C-terminally derivatized peptides" LETTERS IN PEPTIDE SCIENCE, vol. 2, no. 6, 1996, pages 325-331, XP009051616 ISSN: 0929-5666

## Description

### Technical field

The present invention relates to an improved process for the preparation of N⁶-(aminoiminomethyl)-N²-(3-mercapto-1-oxopropyl-L-lysylglycyl-L-α-aspartyl-L-tryptophyl-L-prolyl-L-cysteinamide, cyclic(1→6)-disulfide of formula (1) using solid phase Fmoc-chemistry.

### Background and prior art references of the invention

US Patent No.5318899 describes N⁶-(aminoiminomethyl)-N²-(3-mercapto-1-oxopropyl-L-lysylglycyl-L-α-aspartyl-L-tryptophyl-L-prolyl-L-cysteinamide, cyclic (1→6)-disulfide of the formula (1) as a therapeutic agent for the treatment of, and prevention of, platelet-associated ischemic disorders. It binds to the platelet receptor glycoprotein (GP) of human platelets and inhibits platelet aggregation. Platelet aggregation is mediated by GP complex on the surface of the platelet membrane. It exists on the surface of unstimulated platelets in an inactive form. When platelets are activated by adhesion and the physiological agonists, the GP also becomes activated such that it becomes a receptor for fibrinogen, von Willebrand Factor (vWF), and fibronectin. However, it is the binding of fibrinogen and/or vWF that is believed to be principally responsible for platelet aggregation and thrombus formation *in vivo*. This teaches that substances which specifically inhibit the binding of fibrinogen or vWF to GP, inhibit platelet aggregation and could be candidates for inhibiting thrombus formation *in vivo* (Eric J. Topol, Tatiana V. Byzova, Edward F. Plow and The Lancet; Vol 353; January 16, 1999; pg 227-231). This article describes the compound having platelet aggregation inhibition activity but does not teach the method to synthesize the molecule.

Antagonists of platelet glycoprotein IIb/IIIa have an approved role in reducing the extent of thrombotic complications leading to myocardial damage during percutaneous coronary interventions (PCI).
Specificity and potency of the disintegrin family of antagonists, in particular barbourin, can be mimicked by small, conformationally restrained peptides (Scarborough R. M. et al., Journal of Biological Chemistry vol. 268, no. 2, 1993, 1066-1073). The article describes incorporation of KGD sequence into the peptide, followed by systematic optimization of the cyclic ring size, optimization of secondary hydrophobic binding site interactions, and the derivation of the lysyl side chain functionality of the KGD sequence which resulted in peptide analogues with GP IIb-IIIa selectivity comparable to barbourin that displayed inhibitory potency.

Compound of formula (1) is a disulphide looped cyclic heptapeptide containing six amino acids and one mercaptopropionyl(desamino cysteinyl) residue. The disulfide bridge is formed between the cysteine amide and the mercaptopropionyl moieties. It is known to be produced by solution-phase peptide synthesis and purified by preparative reverse phase liquid chromatography and lyophilized (www.fda.gov/cder/foi/label/1998/207181bl.pdf).

In terms of peptide synthesis methodology, two major synthetic techniques dominate current practice. These are synthesis in solution (homogeneous phase) and synthesis on solid phase (heterogeneous phase). But solution phase route is more cumbersome as compared to the solid phase route as after each coupling the peptide formed has to be isolated, whereas in the solid phase synthesis, the excess reagents and by-products are washed off by simple filtration. In both, the desired peptide compound is prepared by the step-wise addition of amino acid moieties to a building peptide chain.

US patents 5958732 and 5318899 claim about recombinant techniques to synthesize peptides like N⁶-(aminoiminomethyl)-N²-(3-mercapto-1-oxopropyl-L-lysylglycyl-L-α-aspartyl-L-tryptophyl-L-prolyl-L-cysteinamide, cyclic(1→6)-disulfide of the formula (1). The peptide obtained by this recombinant process is modified by solution phase synthesis for conversion of lysine residue to homoarginine residue. These patent documents also claim solid phase synthesis using Boc chemistry and the subject matter of these patents is fundamentally different from the present invention.

Method for systematic C-terminal modification of cystein-containing peptides by solid phase synthesis has been described where cystein is linked to chloromethylated polystyrene resin by its thiol functionality, followed by protection of the N-terminus and derivation of the carboxylic acid to esters or amides (Delaet N. G. J. et al., Letters in Peptide Science, vol.2, no. 6, 1996, 325-331, XP009051616 ISSN: 0929-5666)

As compared to Boc-chemistry, Fmoc-chemistry based synthesis utilizes a mild procedure and because of the base lability of Fmoc group, acid-labile side-chain protecting groups are employed providing orthogonal protection. The rationale for use of protecting groups is that the energy of breaking a bond of a protecting group is lower than any other group.

Patents US5686566, US5686567, US5686569, US5686570 and US5756451 deal with different PAI's in their salt or other forms of the compound of formula (1) but do not teach the process for its preparation using Fmoc solid phase synthesis.

Likewise, patents US5759999, US5786333, US5770564, US5807825, US5807828, US5843897, US5968902, and US5935926 describe the method of treating platelet-associated disorders and the process for the preparation of peptide amide of formula (1) using boc chemistry.

Patents US5344783 and US5851839 deal with methods for selecting and identifying Platelet Aggregation Inhibitors (PAI) and disclose boc chemistry for the preparation of peptide amide of formula (1).

US patent 5780595 claims antibodies specific to PAI's and also discloses boc chemistry for the preparation of the peptide amide of formula (1).

The Fmoc route of synthesis of various other peptides is well-known in prior art and several documents are available for their preparation. However there is a definite need to develop a process for the preparation of compound of formula (1) which is economical, involves minimal steps and also eco-friendly.

As explained earlier, Fmoc-chemistry based synthesis utilises a mild procedure and because of the base lability of Fmoc group, acid-labile side-chain protecting groups are employed providing orthogonal protection. The protecting groups used in Fmoc chemistry are based on the tert-butyl moiety: tert-butyl ethers for Ser, Thr, tert-butyl esters for Asp, Glu and Boc for Lys, His. The trt and acm groups have been used for the protection of Cys. Theguanidine group of Arg and Har is protected by Mtr, Pmc or Pbf. Most of the Fmoc-amino acids derivatives are commercially available. However, a problem exists in the art for the preparation of some amino acid analogs like peptides containing homoarginine as well as cyclic peptide compounds based on disulfide links, because separate operations are required before purifying the end product, which increases expense and may affect final product purity and yield. Fmoc-homoarginine residue if purchased commercially for use in the assembly of the chain becomes expensive. Alternatively in the peptide assembly, the Har unit is built by guanylation of the lysine residue at the α-NH₂, which has been demonstrated to obtain vasopressin analogues for the evaluation of its biological activity (Lindeberg et al, Int. J. Peptide Protein Res. 10,1977,240-244).

WO 03/093302 discloses the synthesis of the peptide of formula (1) using Fmoc-α-nitrogen protected Cα-carboxamide cysteine. It describes the attachment of the first amino acid, cysteine in the precipitated form to the solid support 4-methoxytrityl polystyrene resin through its thiol side chain, followed by removing the α-nitrogen protecting group and assembling the peptide on the said nitrogen. However, the process uses the solid support - 4-methoxytrityl polystyrene resin which is not a common commercial embodiment and also the Fmoc-α-nitrogen protected Cα-carboxamide cysteine is not commercially available. This enables the process having increased number of steps and also expensive with respect to the process of the present invention. The cleavage conditions utilize ethanedithiol, which makes the process highly toxic and non-environment friendly requiring the use of expensive scrubbers. The use of Fmoc-homoarginine residue in the assembly of the chain is mentioned, which if purchased commercially, also makes the process very expensive. Overall, the process claimed in this document is different from the process claimed in the present invention. In addition the process of WO 03/093302 is associated with certain limitations, which has been overcome by providing suitable modifications in the process steps of the present invention.

Thus process of the present invention is an improved and efficient process over the one described in WO03/093302-A2 patent publication as herein mentioned below.
- 1: Does not involve the production of -SH peptide, which is susceptible to aerial oxidation leading to the formation of impurities, which hamper the purification of the final product and yield.
- 2: Precise selection of protecting groups for amino acids to build the peptide chain.
- 3: Activation of carboxylic function of the amino acid using appropriate activating reagent to prevent the racemization of amino acids.
- 4: Efficient process for obtaining disulfide loop in the peptide amide of formula (1) from silver-peptide salt intermediate without isolating -SH peptide.

A considerable number of known, naturally occurring small and medium-sized cyclic peptides as well as some of their synthetic derivatives and analogs possessing desirable pharmacological properties have been synthesized. However, wider medical use is often hampered due to complexity of their synthesis and purification. Therefore, improved methods for making these compounds in simple, lesser steps and at lesser cost are desirable and it is the need of the industry and mankind.

The purity and yield of the peptide are important aspects of any route of synthesis. Yield, represented by the relative content of the pharmacologically active compound in the final product, should be as high as possible. Purity is represented by the degree of presence of pharmacologically active impurities, which though present in trace amounts only, may disturb or even render useless the beneficial action of the peptide when used as a therapeutic agent. In a pharmacological context both aspects have to be considered. As a rule, purification becomes increasingly difficult with larger peptide molecules. In homogeneous (solution) phase synthesis (which is the current method of choice for industrial production of larger amounts of peptides) repeated purification required between individual steps provides a purer product but low yield. Thus, improvements in yield and purification techniques at the terminal stages of synthesis are needed. The present invention is an industrially feasible solid phase synthesis and is a novel process to yield a high purity product with enhanced yield.

Prior art describes the use of HOBt and DIC for activation of amino acids, which leads to the formation of the OBt ester. However, a major drawback in using this procedure is that the preparation of the OBt ester itself takes about 20 min and also the reaction has to be carried out at 0°C. The step-wise introduction of Nα-protected amino acids in SPPS normally involves in situ carboxyl group activation of the incoming amino acid or the use of pre-formed activated amino acid derivatives. In recent years, aminium and phosphonium based derivatives (HBTU, TBTU, Py Boc, and HATU) have become the preferred tools for in situ carboxyl activation. They have been shown to give superior results in terms of both coupling efficiency and suppression of enantiomerization. (Fmoc Solid Phase Peptide Synthesis by Chan W.C. and White P.D., Oxford University Press, 2000, p. 41 - 74) Use of HBTU provides high yield and high purity. It saves time in the activation step with no cooling required. Double coupling is also not required for Mpr(Acm)-OH.

Most of the Fmoc-amino acids derivatives are commercially available. However, a problem exists in the art for the preparation of some amino acid analogs like peptides containing homoarginine as well as cyclic peptide compounds based on disulfide links, because separate operations are required before purifying the end product, which increases expense and may affect final product purity and yield. Fmoc-homoarginine residue if purchased commercially for use in the assembly of the peptide chain becomes very expensive. Alternatively the peptide assembly can be built using lysine followed by guanylation of the lysine residue at the α-NH₂ (Lindeberg et al, Int.J. Peptide Protein Res.10, 1977, 240-244).
Oxidative cyclization of protected or non-protected sulfhydryl groups with formation of disulfide structures is usually carried out as the final synthetic step, the reason being substantial thermal and chemical lability of the disulfide linkage. In few cases it is also carried out before cleavage of the peptide molecule from the solid support. The oxidation of open-chain peptides containing free and/or certain types of protected sulfhydryl groups with iodine in, e.g., methanol or acetic acid is further explained in the CRC Handbook ofNeurohypophyseal Hormone Analogs, Vol. 1, Part 1; Jost, K., et al. Eds., CRC Press, Boca Raton, Fla. 1987, p. 31. Iodine, however, is not without drawbacks as a cyclization agent. For instance, tryptophan moieties present in peptide substrates are at risk of being iodinated, making the balance between full conversion of starting materials and minimizing side reactions a delicate one, which, in turn, impacts product purity. Tam (Tam J.P. et al., 1991, J. Am. Chem. Soc., Vol. 113, p. 6657) has demonstrated that the use of 20 - 50% solutions of DMSO in a variety of buffer systems greatly promotes disulfide bond formation in comparison with other methods such as aerial oxidation. DMSO is also found to greatly reduce and in some instances, suppress completely, the aggregation and precipitation of peptides that occurred using other oxidative procedures. Thus, the yield and purity of the disulfide looped peptide oxidized by DMSO is much higher as compared to other known methods. In the present invention this aspect has been rightfully tackled by not opting for Iodine route for oxidative cyclization. Also in the present invention the silver salt of peptide amide in place of peptide amide containing thiol group is subjected to oxidation without isolation of SH-peptide and eliminating the formation offside products during oxidation reaction. Thus the process steps of deprotection followed by oxidation of guanylated peptide amide adopted in the present invention yields crude peptide amide comprising compound of formula (1) of enhanced purity and yield. Finally purification of the crude peptide result in enhanced yield of the final pure peptide.

Another complicating factor in known routes of synthesis is the possibility of interaction between the desired cyclic disulfide and inorganic sulfur compounds used for reducing excess iodine at the end of the reaction, such as sodium dithionite or sodium thiosulfate. Such reducing sulfur-containing compounds may interact with the disulfide linkage, which is sensitive to nucleophilic attack in general. As the process of the present invention has avoided use of iodine, the resulting products have high purity and related impurities are undetectable.

The process is accomplished in a few easy and simple steps. The use of solid phase synthesis makes the process simpler and the use of Fmoc-chemistry eliminates the use of harsh chemicals like HF, thereby not affecting the product stability. The process eliminates purification of the intermediates, thereby increasing the yield and reducing the cost. Replacement of thiols as scavengers in step (b) and (e) makes the process more environment friendly and economical by not having to use scrubbers for thiols.

The choice of process often dictates the stability of the therapeutic peptide. There has been a long awaited requirement for obtaining peptide of formula (1) which will circumvent the limitations associated with the processes of prior art. Therefore, an industrial process of peptide synthesis containing tryptophan, disulfide loops, ε-NH₂ side chain, etc demands appropriate choice of protecting groups and reaction conditions to build up the peptide chain. This objective has been now successfully achieved by the Applicant developing a process described in entirety in the present application.

**Glossary of terms used in the specification**

| | |
|---|---|
| AA | Amino Acid |
| Acm | Acetamidomethyl |
| ACT | Activator |
| ADP | Adenosine diphosphate |
| AgOTf | Silver trifluoromethane sulfonate |
| Arg | Arginine |
| Asp | Aspartic Acid |
| Boc / boc | tert-butyloxycarbonyl |
| Cys | Cysteine |
| DCM | Dichloromethane |
| DEP | Deprotection reagent |
| DMF | Dimethyl Formamide |
| DMSO | Dimethyl sulphoxide |
| DTT | Dithiothreitol |
| EDT | Ethane dithiol |
| Fmoc | 9-fluorenylmethyloxycarbonyl |
| Glu | Glutamic acid |
| Gly | Glycine |
| HBTU | 2-(1H-Benzotriazolel-yl)-1,1,3,3-tetramethyluronium hexafluorophosphate |
| HF | Hydrogen Fluoride |
| HIC | Hydrophobic Interaction Chromatography |
| His | Histidine |
| IEC | Ion Exchange Chromatography |
| LC-MS | Liquid Chromatography-Mass Spectroscopy |
| Lys | Lysine |
| Mpr | Mercaptopropionic Acid |
| Mtr | 4-methoxy-2,3,6-trimethylbenzenesulfonyl |
| NMM | N-methyl morpholine |
| Obut | O-*t*-butyl |
| Pbf | 2,2,4,6,7-pentamethyldihydrobenzofuran-5-sulfonyl |
| Pmc | 2,2,5,7,8-pentamethylchroman-6-sulfonyl |
| PPP | Platelet poor plasma |
| Pro | Proline |
| PRP | Platelet rich plasma |
| RP-HPLC | Reverse Phase High Performance Liquid Chromatography. |
| RV | Reaction Vessel |
| Ser | Serine |
| SOLV | Solvent |
| SP | Synthetic Peptide |
| TEA | Triethylamine |
| TFA | Trifluoroacetic acid |
| Thr | Threonine |
| TIS | Triisopropylsilane |
| Trp | Tryptophan |
| Trt | Trityl |

### Objects of the Invention

The main object of the present invention is to provide an improved process to obtain N⁶-(aminoiminomethyl)-N²-(3-mercapto-1-oxopropyl-L-lysylglycyl-L-α-aspartyl-L-tryptophyl-L-prolyl-L-cysteinamide, cyclic(1-→6)-disulfide of formula (1).

Another object of the present invention is to disclose a process for obtaining high yield and high purity of peptide amide of formula (1)

Yet another objective of the present invention is to disclose a process of solid phase synthesis of peptide amide of formula (1) by using Fmoc chemistry.

Still another object of the present invention is to disclose a process for the production of peptide amide of formula (1), having lesser number of steps as compared to solution phase synthesis.

Yet another object of the present invention is to design a process for the production of peptide amide of formula (1), which is devoid of limitations associated with prior art solid phase synthesis of compound of formula (1).

Still yet another object is to provide a process for preparing small and medium-size peptide amides containing a disulfide moiety having enhanced purity.

Still yet another object of the present invention is to select appropriate protecting groups and reagents to minimize the formation of accompanying impurities in process steps, thereby enhancing the yield and reducing the cost.

### Summary of the Invention

The present invention relates to an improved process for the preparation of N⁶-(aminoiminomethyl)-N²-(3-mercapto-1-oxopropyl-L-lysylglycyl-L-α-aspartyl-L-tryptophyl-L-prolyl-L-cysteinamide, cyclic(1→6)-disulfide of formula (1), which involves assembling amino acid residues and a thioalkyl carboxylic acid with an appropriate protecting groups on a solid phase resin, cleaving the peptide amide thus obtained from the resin with concomitant removal of side chain protecting groups except Acm protecting group of thiol moiety to obtain peptide amide of formula (3), converting lysine residue of peptide amide of formula (3) having protected thiol group to homoarginine residue by guanylation, followed by simultaneous deprotection, obtaining silver peptide of formula (5) and oxidation of silver peptide to obtain crude peptide amide of formula (1) and finally subjecting to chromatographic purification. The described process is simple, easy, environment friendly and cost effective.

### Brief description of figures and Table

**Fig. 1****:** Analytical RP-HPLC elution profile of HBTU- crude peptide amide from resin (Column: PEP 300; C-18; 5µ; 150 X 3 mm; Flow rate: 0.5ml/min; Injection vol: 20µl; Solvent System: A: 0.1% TFA, B: 100% Acetonitrile).
**Fig. 2****:** Analytical RP-HPLC elution profile of DIC- crude peptide amide from resin (Column: PEP 300; C-18; 5µ; 150 X 3 mm; Flow rate: 0.5ml/min; Injection vol: 20µl; Solvent System: A: 0.1% TFA, B: 100% Acetonitrile).
**Fig. 3****:** Analytical RP-HPLC elution profile of crude guanylated peptide amide (Column: PEP 300; C-18; 5µ; 150 X 3 mm; Flow rate: 0.5m/min; Injection vol: 20µl; Solvent System: A: 0.1% TFA, B: 100% Acetonitrile).
**Fig. 4****:** Analytical RP-HPLC elution profile of SH peptide amide (Column: PEP 300; C-18; 5µ; 150 X 3 mm; Flow rate: 0.5ml/min; Injection vol: 20µl; Solvent System: A: 0.1% TFA, B: 100% Acetonitrile). **Peak A-** crude SH peptide amide.
**Fig. 5****:** Analytical RP-HPLC elution profile of crude cyclic peptide amide (Column: PEP 300; C-18; 5µ; 150 X 3 mm; Flow rate: 0.5ml/min; Injection vol: 20µl; Solvent System: A: 0.1% TFA, B: 100% Acetonitrile); **Peak A-** crude cyclic peptide amide.
**Fig. 6****:** Preparative RP-HPLC purification elution profile of crude cyclic peptide amide (Column: Phenomenex Luna; C-18(2); 10µ; 250 X 50 mm; Flow rate: 50ml/min; Solvent System: A: 0.1% TFA, B: 100% Methanol).
**Fig. 7****:** Analytical RP-HPLC elution profile of purified cyclic peptide amide (Column: PEP 300; C-18; 5µ; 150 X 3 mm; Flow rate: 0.5ml/min; Solvent System: A: 0.1% TFA, B: 100% Acetonitrile); **Peak A-** purified peptide amide.
**Fig.8****:** MS Analysis of the pure peptide amide showing the mass to be 832 and impurity to be 903
**Table 1:** Inhibition of ADP induced aggregation by synthesized peptide amide of formula (1).

### Detailed Description of the Invention

In accordance, the present invention provides a. process for the preparation of a peptide amide N⁶-(aminoiminomethyl)-N²-(3-mercapto-1-oxopropyl-L-lysylglycyl-L-α-aspartyl-L-tryptophyl-L-prolyl-L-cysteinamide, cyclic(1→6)-disulfide of formula (1) on a solid phase, the said process comprising steps of, 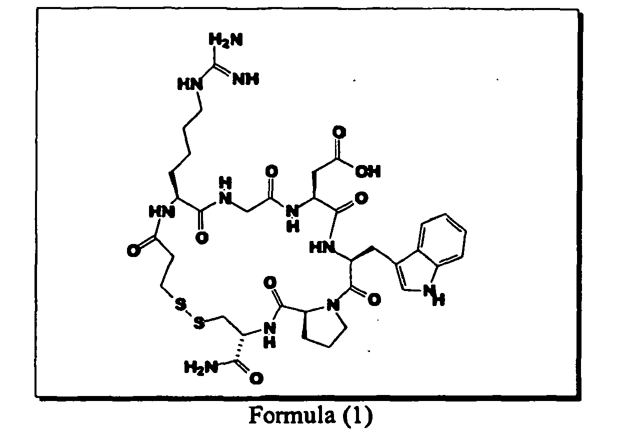
a) assembling a peptide chain comprising of six amino acids and a thioalkyl carboxylic acid in a required sequence on a solid support resin by coupling to directly join one another by peptide bonds to obtain peptide of formula (2);

   (Acm)Mpr-Lys(Boc)-Gly-Asp(Obut)-Trp-Pro-Cys(Acm)-Resin Formula (2)
b) capping the free amino groups after each coupling of step (a) with acetic anhydride;
c) cleaving and deprotecting, all groups except Acm group in the peptide of step (b) from the solid support resin to obtain peptide amide of formula (3);

   (Acm)Mpr-Lys-Gly-Asp-Trp-Pro-Cys(Acm)-CONH₂ Formula (3);
d) guanylating the peptide amide of Formula (3) at ε-lysine-NH₂ in an organic solvent followed by precipitating with an another solvent to obtain peptide amide of formula (4);

   (Acm)Mpr-Homoarg-Gly-Asp-Trp-Pro-Cys(Acm)-CONH₂ Formula (4)
e) treating the peptide amide of Formula (4) with a heavy metal salt in an appropriate solvent, followed by precipitating using an organic solvent to obtain the heavy metal-peptide salt of formula (5); 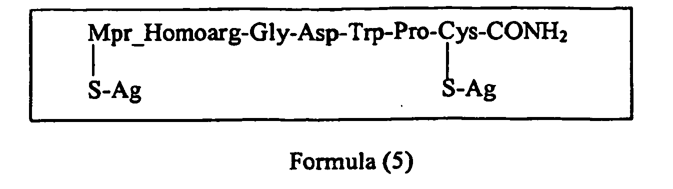
f) treating the heavy metal-peptide salt of step (e) with an appropriate nucleophilic reagent to obtain the crude peptide amide of formula (1); and
g) purifying the crude peptide amide of step (f) by chromatographic techniques.

An embodiment of the present invention involves reaction of amino and carboxylic equivalent of compounds to form the said peptide bond.

Another embodiment of the present invention provides C-terminal of ,the protected first amino acid bound to a solid phase resin through a linker to obtain a solid phase bound amino acid.

Yet another embodiment of the present invention uses as solid support any amide resin, preferably Rink Amide Resin.

Still another embodiment of the present invention uses first protected amino acid as thiol protected Fmoc cysteine.

Yet another embodiment of the present invention uses HBTU as the coupling agent.

Still yet another embodiment of the present invention provides a cleavage of the resin with the linker leading to release of assembled peptide amide.

Yet another embodiment of the present invention provides peptide amide compound of formula (1) which is a compound joined to each of the terminal functionalities by a peptide bond and wherein each terminal functionality is an amino or carboxylic acid group.

Still another embodiment of the present invention uses amino acids selected from the group consisting of Cys, Pro, Trp, Asp, Lys, Gly, Arg, Har, Leu, Glu.

An embodiment of the present invention uses thioalkyl carboxylic acid 2-thiopropionic acid.

Another embodiment of the present invention provides the use of protecting groups for amino function of an amino acid as Fmoc or Boc.

Yet another embodiment of the present invention provides the use of carboxyl function as unprotected or protected O-tBu ester.

Still another embodiment of the present invention uses the protecting group for thiol-function as Acm group.

Still yet another embodiment of the present invention provides cleavage of the peptide from solid support resin using the reagents TFA, TIS, EDT, DCM, Phenol and water in a defined ratio, preferably TFA(85-98%) : TIS(0-5%) : H₂O(0-5%) : EDT(0-5%): Phenol(0-5%), more preferably TFA(94.5-95.5%) : TIS(0-2.5%) : H₂O(0-3%) : EDT(0-2.5%).

Another embodiment of the present invention utilizes an organic solvent for guanylation selected from a group consisting of DMF, ethanol and methanol.

Yet another embodiment of the present invention the guanylation of peptide is performed preferably by using the solvent DMF.

Still another embodiment of the present invention the precipitation of the peptide amide of formula (4) is performed using a solvent selected from the group consisting of acetone, acetonitrile, methanol, ethers, pentane, hexane and mixture thereof.

Still yet another embodiment of the present invention the precipitation is preferably performed using acetonitrile.

Another embodiment of the present invention) the purification of the peptide amide of formula (4) can be achieved by RP-HPLC.

Yet another embodiment of the present invention the peptide amide of formula (1) obtained has purity of more than 99%.

Still yet another embodiment of the present invention the preparation of the peptide amide of formula (1) by solid phase synthesis is carried out using Fmoc chemistry.

Further embodiment of the present invention uses heavy metal salt for removal of Acm selected from thallium trifluoromethane sulphonate, mercuric acetate or silver trifluoromethane sulphonate, preferably silver trifluoromethane sulphonate.

In another embodiment of the present invention the heavy metal peptide salt is obtained by preferably treating peptide amide of formula (4) with silver trifluoromethane sulphonate in TFA.

Yet in another embodiment of the present invention the precipitation of the heavy metal-peptide salt of Formula (5) is preferably carried out using an etheral solvent and more preferably disopropyl ether.

Still in another embodiment of the present invention the heavy metal-peptide salt may be treated with HCl and DMSO to simultaneously remove the heavy metal and to oxidize the resulting peptide to yield crude peptide amide of formula (1).

Still yet in another embodiment of the present invention the crude peptide amide of formula (1) may be purified by RP-HPLC.

In another embodiment of the present invention the purification of crude peptide amide of formula (1) is preferentially performed by RP-HPLC using C-4, C-8 or C-18 silica or polymer reverse phase columns using methanol and/or acetonitrile in combination with aqueous TFA(0-0.5%) as mobile phase

Still another embodiment of the present invention uses methanol (AR grade) for purification of crude peptide amide enabling the process inexpensive.

Yet another embodiment of the present invention provides process for preparation of an intermediate peptide of formula (2) as given under:

(Acm)Mpr-Lys(Boc)-Gly-Asp(Obut)-Trp-Pro-Cys(Acm)-Resin Formula (2)

Still another embodiment of the present invention provides process for preparation of an intermediate peptide amide of formula (3) as given under:

(Acm)Mpr-Lys-Gly-Asp-Trp-Pro-Cys(Acm)-CONH₂ Formula (3)

Still yet another embodiment of the present invention provides process for preparation of a peptide amide of formula (4) as given below:

(Acm)Mpr-Homoarg-Gly-Asp-Trp-Pro-Cys(Acm)-CONH₂ Formula (4)

Yet another embodiment of the present invention provides process for preparation of an intermediate peptide amide silver salt of formula (5) as given under: 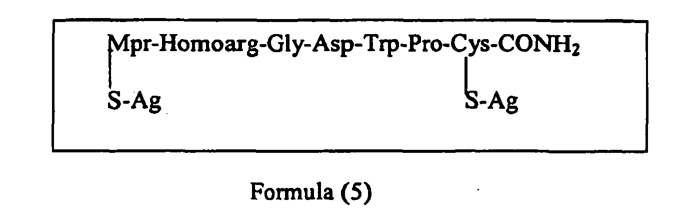 The following examples are illustrative of the present invention and not to be construed to limit the scope of the invention.

### EXAMPLES

### EXAMPLE (1) Chemical synthesis of linear peptide

(Acm)Mpr-Lys(Boc)-Gly-Asp(OBut)-Trp-Pro-Cys(Acm)-Resin Formula (2)

### General Procedure:

The assembly of the peptide chain is carried out in the following manner. The resin is transferred to the RV of the peptide synthesizer [CS936, CS BIO, Calif. Peptide Synthesizer] and the linear peptide is assembled on it using 1.5 - 4.0 times mole excess amino acid derivatives, on the peptide synthesizer. The first amino acid, Fmoc-Cys (C), is coupled to the resin by deprotecting the Fmoc-group on the resin, followed by activating the Fmoc-Cys(C) by HBTU in the presence of NMM. For coupling of the next amino acid, Proline, the α-nitrogen of the first amino acid i.e. Fmoc-Cys(C), is deprotected followed by activating the Fmoc-Pro by HBTU in the presence of NMM. This process is repeated with all the amino acids till the entire linear peptide chain is assembled on the solid support. The Mpr is assembled at the end. Each coupling is carried out for a time range of 45-90 min. The coupling steps are followed by capping with acetic anhydride for 30-60 min. After the coupling are complete, the resin is washed with organic solvent/s which may be selected from the range of DMF, N-methyl pyrrolidone or DCM, preferably DMF followed by DCM, and then dried under vacuum. The linear peptide of formula (2) is obtained.

The peptide was synthesized as peptide amide by solid phase peptide synthesis technology on rink amide resin using Fmoc chemistry.

| | |
|---|---|
| **Instrument** | CS936, CS BIO, Calif. Peptide synthesizer |
| **Resin** | Rink amide resin (0.65mm/g) |
| **Activator** | HBTU/0.4M NMM |
| **Solvent** | Dimethyl Formamide |
| **Deprotection** | 20%Piperidine |

The resin (15.38g-rink amide, 10 mmole) was transferred to the RV of the CS936 and swollen in DMF.
Synthesis of Fmoc Cys(Acm)-resin by coupling of Fmoc-Cys(Acm)/HBTU to the resin. The pre-swollen resin (10mmole) was washed twice with DMF followed by removal of Fmoc by treatment with 20% piperidine twice. The resin was washed 6 times with DMF. Fmoc Cys(Acm)(20mmoles) and HBTU (equimole to amino acid) were dissolved in 0.4M NMM and added to the resin. Coupling was carried out for 60min under optimized stirring. The resin was washed once again with DMF thrice. After the coupling, the free amino groups were capped by acetic anhydride (2.5M) for 45 min followed by washing with DMF three times. This HBTU process is a one-step process wherein ester is not isolated.

The synthesis cycle was programmed as follows:

| **Step** | **Reagent** | **Time** | **Repeat** | **Activity** |
|---|---|---|---|---|
| 1 | SOLV | 10min | X3 | WASHES RESIN |
| 2 | DEP | 5min | X2 | DEP N-TERMINUS |
| 3 | SOLV | 30sec | X6 | WASHES RESIN |
| 4 | ACT | 30sec | X1 | DISSOLVES Fmoc-Cys (Acm)/HBTU |
| 5 | AA | 45min | X1 | Fmoc-Cys (Acm) COUPLING |
| 6 | SOLV | 30sec | X3 | WASHES RESIN |

**(ii) Synthesis of Fmoc-Pro-Cys(Acm)-resin** by coupling Fmoc-Pro/HBTU to Fmoc-Cys(Acm)-resin. The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5mm | X2 | DEP N-TERMINUS |
| 3 | SOLV | 30sec | X6 | WASHES RESIN |
| 4 | ACT | 30sec | X1 | DISSOLVES Fmoc-Pro/HBTU |
| 5 | AA | 45min | X1 | COUPLING Fmoc-Pro |
| 6 | SOLV | 30sec | X3 | WASHES RESIN |

**(iii)Synthesis of Fmoc-Trp-Pro-Cys(Acm)-resin** by coupling Fmoc-Trp/HBTU to Fmoc-Pro- Cys(Acm)-resin. The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | ACT | 30sec | X1 | DISSOLVES Fmoc-Trp/HBTU |
| 5 | AA | 45min | X1 | COUPLING Fmoc-Tcp |
| 6 | SOLV | 30sec | X3 | WASHES RESIN |

**(iv)Synthesis of Fmoc-Asp(Obut)-Trp-Pro-Cys(Acm)-resin** by coupling Fmoc-Asp (Obut)/HBTU to Fmoc-Trp-Pro- Cys(Acm)-resin. The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | ACT | 30sec | X1 | DISSOLVES Fmoc-Asp(Obut)/HBTU |
| 5 | AA | 45min | X1 | COUPLING Fmoc-Asp(Obut) |
| 6 | SOLV | 30sec | X3 | WASHES RESIN |

**(v) Synthesis of Fmoc-Gly-Asp (Obut)-Trp-Pro-Cys(Acm)-resin** by coupling Fmoc-Gly/HBTU to Fmoc-Asp(Obut)-Trp-Pro-Cys(Acm)-resin . The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | ACT | 30sec | X1 | DISSOLVES Fmoc-Gly/HBTU |
| 5 | AA | 45min | X1 | COUPLING Fmoc-Gly |
| 6 | SOLV | 30sec | X3 | WASHES RESIN |

**(vi) Synthesis of Fmoc-Lys(Boc)-Gly-Asp(Obut)Trp-Pro-Cys(Acm)-resin** by coupling Fmoc-Lys(Boc)/HBTU to Fmoc -Gly-Asp(Obut)-Trp-Pro-Cys(Acm)-resin . The reaction was carried out as in step 1.The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | ACT | 30sec | X1 | DISSOLVES Fmoc-Lys(Boc)/HBTU |
| 5 | AA | 45min | X1 | COUPLING Fmoc-Lys(Boc) |
| 6 | SOLV | 30sec | X3 | WASHES RESIN |

**(vii) Synthesis of Mpr(Acm)-Lys(Boc)-Gly-Asp(Obut)-Trp-Pro-Cys(Acm)-resin** by coupling Mpr(Acm)/HBTU to Fmoc-Lys(Boc)-Gly-Asp(Obut)-Trp-Pro-Cys(Acm)-resin. The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | ACT | 30sec | X1 | DISSOLVES Mpr(Acm)/HBTU |
| 5 | AA | 45min | X1 | COUPLING Mpr(Acm) |
| 6 | SOLV | 30sec | X3 | WASHES RESIN |

### EXAMPLE (2) Chemical synthesis of linear peptide

(Acm)Mpr-Lys(Boc)-Gly-Asp(OBut)-Trp-Pro-Cys(Acm)-Resin Formula (2)

### General Procedure:

The assembly of the peptide chain is carried out in the following manner. The resin is transferred to the RV of the peptide synthesizer [PS3, Protein Technologies, Peptide Synthesizer] and the linear peptide is assembled on it using 1.5 - 4.0 times mole excess amino acid derivatives, on the peptide synthesizer. The first amino acid, Fmoc-Cys (C), is coupled to the resin by deprotecting the Fmoc-group on the resin, followed by activation of Fmoc-Cys(C). Fmoc-Cys(C) (1.3 mmole) and HOBt (2.6 mmole) were dissolved in DMF (5.0ml) and cooled to less than 10°C in an ice bath. DIC (1.74 mmole) was added to the reaction mixture as a single aliquot. The mixture was then agitated for 6 minutes before being charged to the damp resin in the reaction vessel. The coupling reaction takes place for 60mins.

For coupling of the next amino acid, Proline, the α-nitrogen of the first amino acid i.e. Fmoc-Cys(C), is deprotected. This is followed by activation of Fmoc-Pro by DIC/HOBt in cold conditions as described above and then transfer of this mixture to the reaction vessel. This process is repeated with all the amino acids till the entire linear peptide chain is assembled on the solid support. The Mpr is assembled at the end. Each coupling is carried out for a time range of 45-90 min. Coupling of Mpr is repeated. The coupling steps are followed by capping with acetic anhydride for 30-60 min. After the coupling are complete, the resin is washed with organic solvent/s which may be selected from the range of DMF, N-methyl pyrrolidone or DCM, preferably DMF followed by DCM, and then dried under vacuum. The linear peptide of formula (2) is obtained.

The peptide was synthesized as peptide amide by solid phase peptide synthesis technology on rink amide resin using Fmoc chemistry.

| | |
|---|---|
| **Instrument** | **PS3, Protein Technologies, Peptide synthesizer** |
| **Resin** | Rink amide resin (0.65mm/g) |
| **Activator** | DIC/HOBT |
| **Solvent** | Dimethyl Formamide |
| **Deprotection** | 20%Piperidine |

The resin (1g-rink amide, 0.65 mmole) was transferred to the RV of the PS3 and swollen in DMF.

Synthesis of Fmoc Cys(Acm)-resin by coupling of activated Fmoc-Cys(Acm) to the resin. The pre-swollen resin (0.65mmole) was washed twice with DMF followed by removal of Fmoc by treatment with 20% piperidine twice. The resin was washed 6 times with DMF. Fmoc Cys(Acm)(1.3mmoles) and HOBt (2.6 mmole) were dissolved in DMF (5.0ml) and cooled to less than 10°C in an ice bath. DIC (1.74 mmole) was added to the reaction mixture as a single aliquot. The mixture was then agitated for 6 minutes before being charged to the damp resin. Coupling was carried out for 60min under optimized stirring. The resin was washed once again with DMF thrice. After the coupling, the free amino groups were capped by acetic anhydride (2.5M) for 45 min followed by washing with DMF three times. This DIC/HOBt process is a manual and multistep process.
The synthesis cycle was programmed as follows:

| **Step** | **Reagent** | **Time** | **Repeat** | **Activity** |
|---|---|---|---|---|
| 1 | SOLV | 10min | X3 | WASHES RESIN |
| 2 | DEP | 5min | X2 | DEP N-TERMINUS |
| 3 | SOLV | 30sec | X6 | WASHES RESIN |
| 4 | Manual addition of activated Fmoc amino acid. | | | |
| 5 | AA | 45min | X1 | Fmoc-Cys (Acm) COUPLING |
| 6 | SOLV | 30sec | X3 | WASHES RESIN |

**(ii) Synthesis of Fmoc-Pro-Cys(Acm)-resin** by coupling activated Fmoc-Pro to Fmoc- Cys(Acm)-resin. The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | Manual addition of activated Fmoc amino acid. | | | |
| 5 | AA | 45min | X1 | COUPLING Fmoc-Pro |
| 6 | SOLV | 30sec | X3 | WASHES RESIN |

**(iii)Synthesis of Fmoc-Trp-Pro-Cys(Acm)-resin** by coupling activated Fmoc-Trp to Fmoc-Pro- Cys(Acm)-resin. The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | Manual addition of activated Fmoc amino acid. | | | |
| 5 | AA | 45min | X1 | COUPLING Fmoc-Trp |
| 6 | SOLV | 30sec | X3 | WASHES RESIN |

**(iv)Synthesis of Fmoc-Asp(Obut)-Trp-Pro-Cys(Acm)-resin** by coupling Fmoc-Asp to Fmoc-Trp-Pro- Cys(Acm)-resin. The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | Manual addition of activated Fmoc amino acid. | | | |
| 5 | AA | 45min | X1 | COUPLING Fmoc-Asp(Obut) |
| 6 | SOLV | 30sec | X3 | WASHES RESIN |

**(v) Synthesis of Fmoc-Gly-Asp (Obut)-Trp-Pro-Cys(Acm)-resin** by coupling Fmoc-Gly to Fmoc-Asp(Obut)-Trp-Pro-Cys(Acm)-resin . The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | Manual addition of activated Fmoc amino acid. | | | |
| 5 | AA | 45min | X1 | COUPLING Fmoc-Gly |
| 6 | SOLV | 30sec | X3 | WASHES RESIN |

**(vi)Synthesis of Fmoc-Lys(Boc)-Gly-Asp(Obut)-Trp-Pro-Cys(Acm)-resin** by coupling Fmoc-Lys(Boc) to Fmoc -Gly-Asp(Obut)-Trp-Pro-Cys(Acm)-resin. The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | Manual addition of activated Fmoc amino acid. | | | |
| 5 | AA | 45min | X1 | COUPLING Fmoc-Lys(Boc) |
| 6 | SOLV | 30sec | X3 | WASHES RESIN |

**(vii) Synthesis of Mpr(Acm)-Lys(Boc)-Gly-Asp(Obut)-Trp-Pro-Cys(Acm)-resin** by coupling Mpr(Acm) to Fmoc-Lys(Boc)-Gly-Asp(Obut)-Trp-Pro-Cys(Acm)-resin. The reaction was carried out as in step 1. The synthesis cycle was programmed as follows:

| **Step** | **Reagent** | **Time** | **Repeat** | **Activity** |
|---|---|---|---|---|
| 1 | SOLV | 30sec | X3 | WASHES RESIN |
| 2 | DEP | 5min | X2 | DEP N-TERMINUS |
| 3 | SOLV | 30sec | X6 | WASHES RESIN |
| 4 | Manual addition of activated Fmoc amino acid. | | | |
| 5 | AA | 45min | X1 | COUPLING Mpr(Acm) |
| 6 | SOLV | 30sec | X3 | WASHES RESIN |

**In the synthesis coupling of Mpr(Acm) had to be carried out twice to complete the coupling reaction.**

### EXAMPLE (3) CLEAVAGE OF THE PEPTIDE FROM THE RESIN TO YIELD PEPTIDE AMIDE (Acm)Mpr-Lys-Gly-Asp-Trp-Pro-Cys(Acm)-CONH₂ (Formula (3))

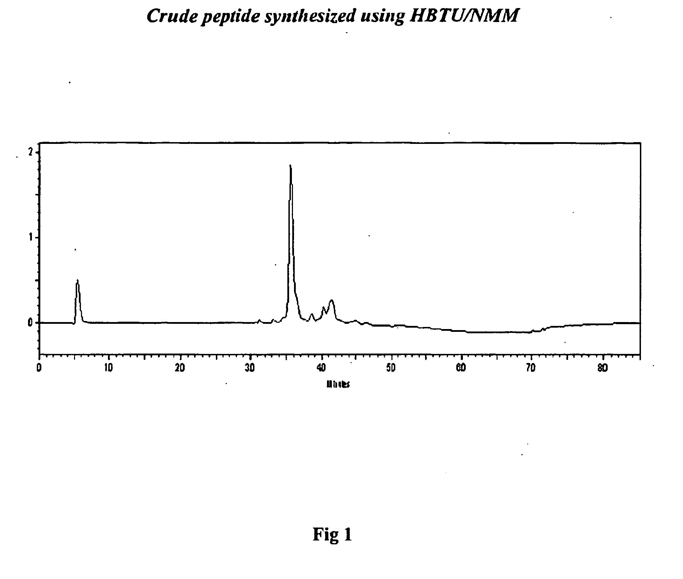

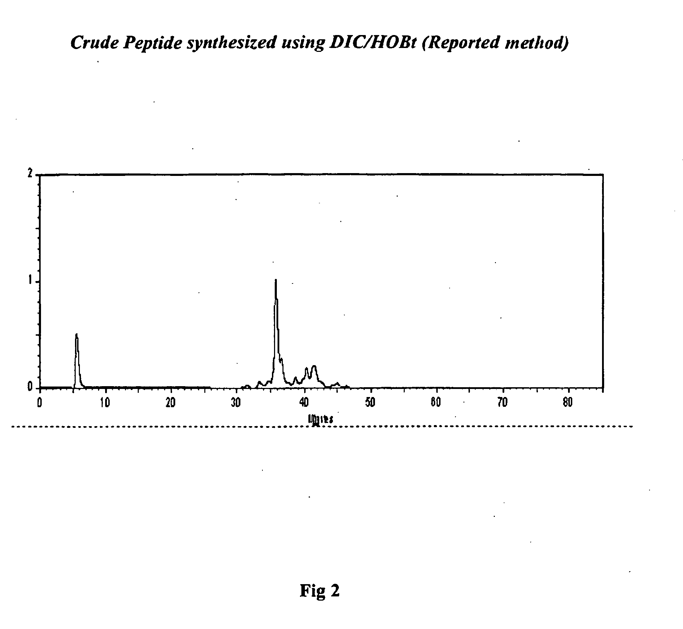

The assembled peptide resin (from Example 1 or 2) is treated with 500 ml of cleavage cocktail consisting of TFA (95%): TIS(2.5%) : H₂O(2.5%) : EDT(O%) : Phenol (0%) for 2 hrs at R.T in CS936. The reaction mixture is filtered through RV, and TFA was evaporated on Rotavap. Precipitation of the peptide was carried out at - 20°C by addition of 300 ml of cold di isopropyl ether with constant stirring. The crude peptide precipitate in the solvent is let to stand at -20°C for 10 hrs. The peptide amide was isolated by filtering through Whatman paper no. 5, followed by cold solvent wash (100ml x 3) to remove the scavengers used in the cleavage cocktail. The crude peptide amide precipitate is dried under vacuum over P₂O₅, and characterized by RP-HPLC (Fig.1 and 2).

| **Example 1** | **Example 2** |
|---|---|
| **Yield: 58.73** | **Yield: 48.73** |
| **% purity of peptide: 90%** | **%purity of peptide: 79.68%** |

### EXAMPLE (4) GUANYLATION OF CRUDE PEPTIDE AMIDE TO YIELD (Acm)Mpr-Homoarg-Gly-Asp-Trp-Pro-Cys(Acm)-CONH₂ (formula (4))

The peptide amide (1g, 1.157mmole) is dissolved in 15 ml of DMF, the pH adjusted to 9.0 with TEA. The reagent 3,5-dimethylpyrazole-1-carboxamidine nitrate (931.5mg) in DMF (15ml) is added to the peptide amide. The reaction mixture is stirred at 30°C for 4 days with multiple additions of one time excess of reagent 3,5-dimethylpyrazole-1-carboxamidine nitrate.

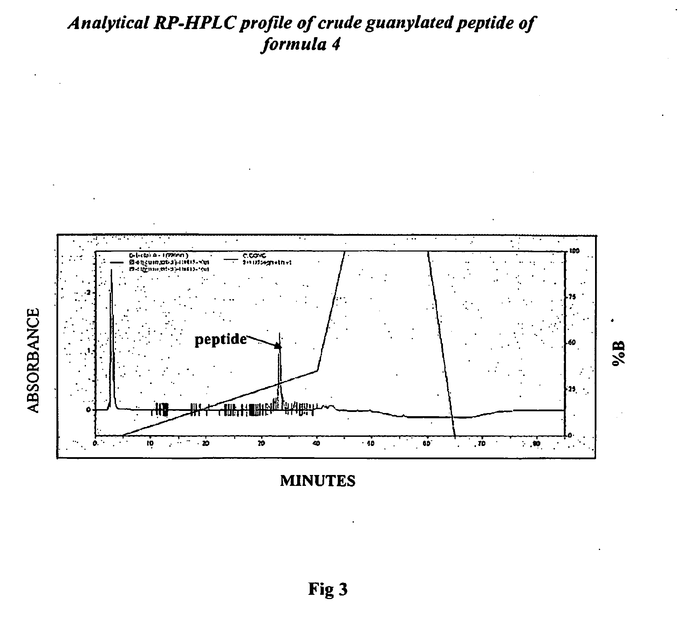

The peptide amide is precipitated from the reaction mixture by the addition of 280ml of acetonitrile (pH adjusted to 8.0 with TEA). The mix is further kept at -20°C for 10 hrs. It is filtered through Whatman no. 5 filter paper and washed with acetonitrile (pH 8.0) 3 times, followed by plain acetonitrile to neutralize the pH. The precipitate is dried under high vacuum overnight. Yield: 85%. The peptide amide was characterized by RP-HPLC (Fig.3).

### EXAMPLE (5) DE-ACM OF THE GUANYLATED PEPTIDE AMIDE FOLLOWED BY OXIDATION TO YIELD

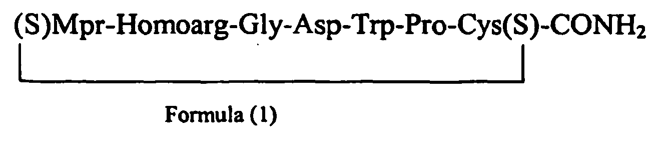

TFA (134.9ml) and anisole (2.7ml) are mixed, cooled in ice, added to 658 mg of pre-cooled peptide amide from example 4 and saturated with nitrogen. This is followed by addition of AgOTf (3.47g) and stirred for 2hrs in an ice bath. TFA is removed under high vacuum and silver salt of the peptide was precipitated by addition of diisopropyl ether (∼400ml). The reaction mixture is filtered through G-4 sintered funnel and precipitate (silver-peptide) is re-suspended in diisopropyl ether (60ml x3), washed as above and dried over P₂O₅ under vacuum.

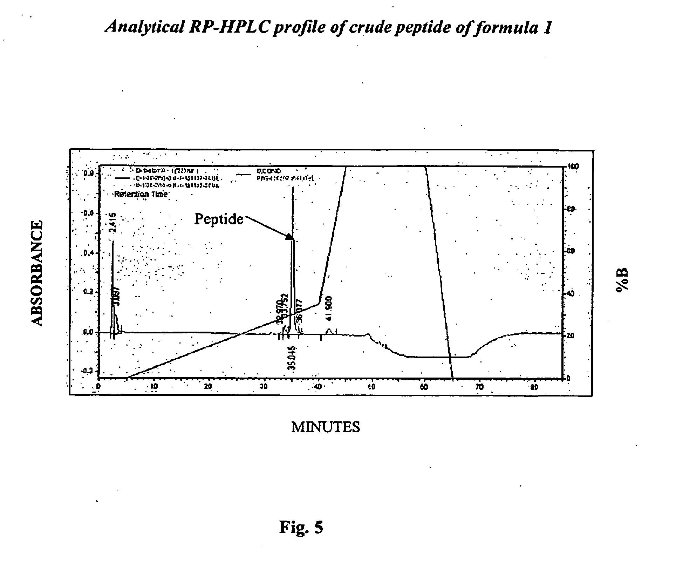

The oxidation silver peptide is carried out by dissolving 10 mg of the silver-peptide salt in 15.6ml of 50% DMSO / IM HCl in ice-cold condition. The reaction mixture is stirred for 3 hrs at 25°C. The precipitate is filtered through a G-4 sintered funnel or Hyflo bed to remove silver chloride. The filtrate is checked for completion of oxidation (Fig. 5).On completion of the reaction crude peptide of formula (1) is obtained. Percentage purity: 85%

### EXAMPLE (6) PURIFICATION OF S-S PEPTIDE AMIDE

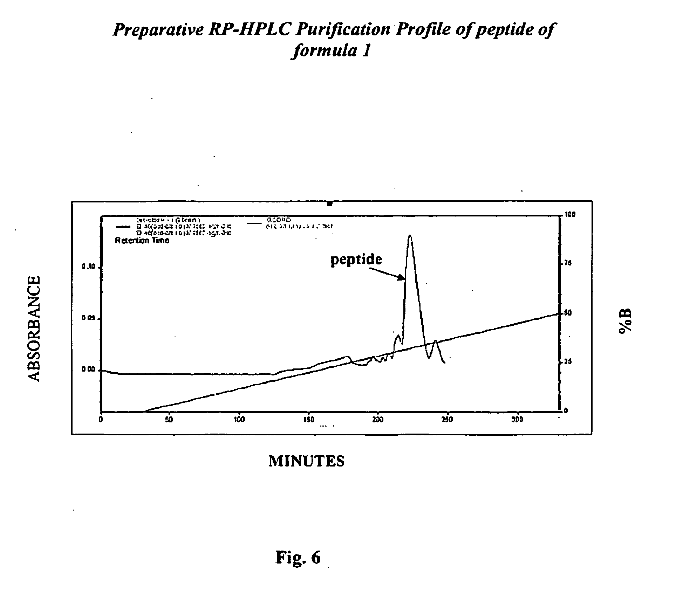

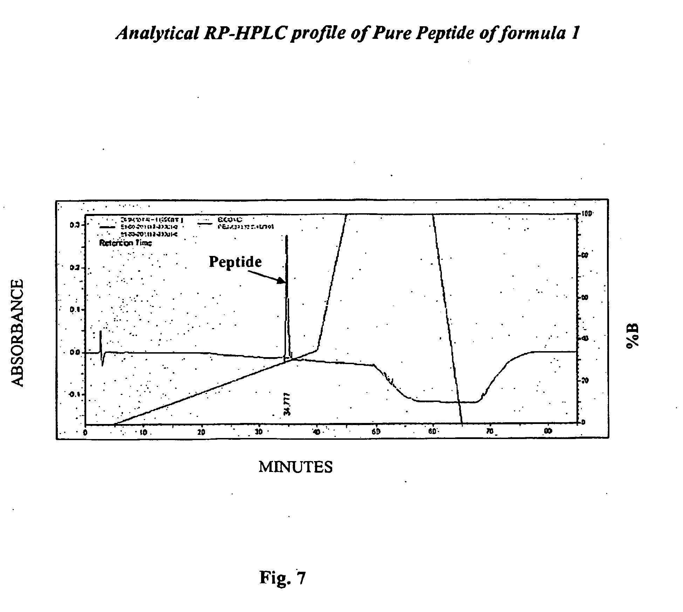

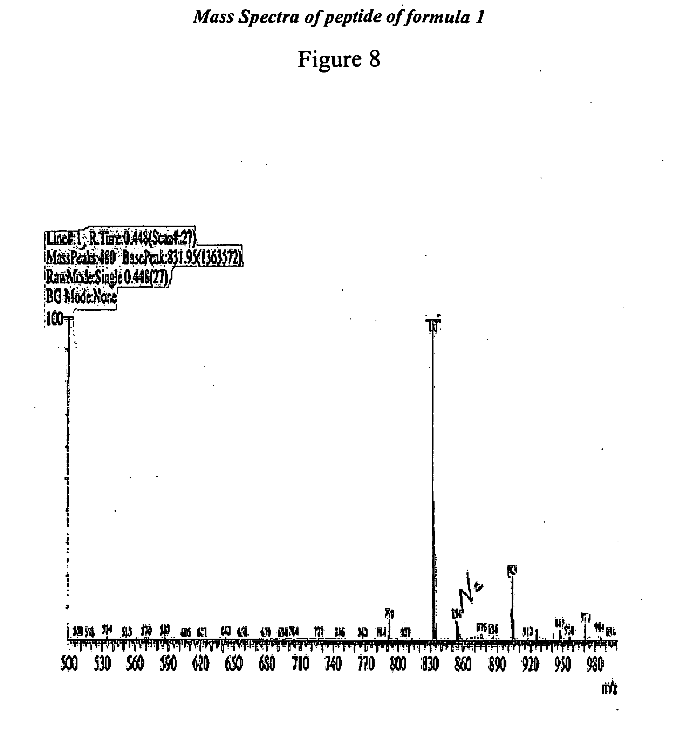

The crude disulfide looped peptide amide of formula (1) is loaded on to prep C-18 column (50 x 250mm, 100Å). The peptide amide is purified by using aqueous TFA (0.1%) and methanol in a gradient program (Fig. 6). This is followed by an isocratic run using the above said solvent systems on a Shimadzu preparative HPLC System consisting of a controller, 2 LC8A pumps, UV-Vis detector. The purified peptide amide of formula (1) is analysed by analytical RP-HPLC (Fig. 7). The mass is determined by Mass Spectrophotometer (Fig. 8).

### EXAMPLE (7): PURIFICATION OF S-S PEPTIDE AMIDE

The purification was carried out in the same manner as Example 5, except that Acetonitrile was used instead of methanol to obtain peptide amide of formula (1).

### EXAMPLE (8): DE-ACM OF THE GUANYLATED PEPTIDE AMIDE USING MERCURIC (II) ACETATE

Same as in Example (4), except that the Acm group protection of cysteineis removed from the guanylated peptide amide by treatment with mercury (II) acetate.

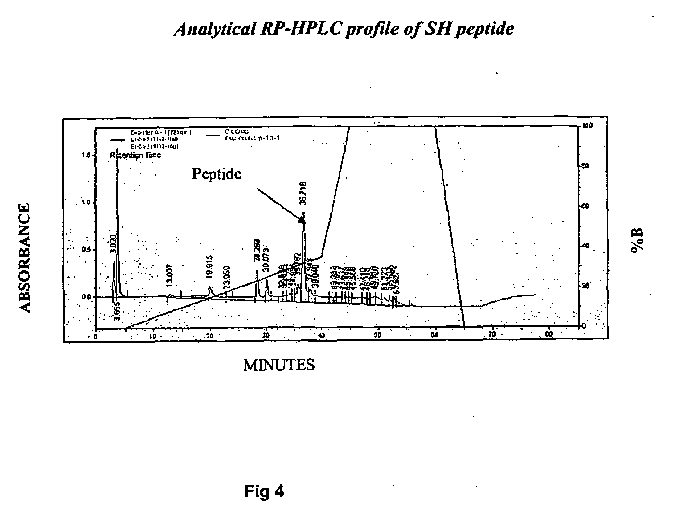

The peptide amide (13.4mg) estimated by Lowry's method, of Cys-Acm) is dissolved in 400µl of acetic acid (10%). Ten times excess of mercury (II) acetate (82.96mg) is added to it, the reaction mass vortexed and kept at R.T. for 5 hrs. 100 times excess of β-mercaptoethanol (181.37µl) is added, the solution vortexed and let to stand overnight at room temperature. The reaction mixture is centrifuged for 4min, and supernatant collected. The precipitate is extracted with 400µl x 3 of 10% acetic acid by centrifugation. The filtrates are pooled and percentage purity determined by RP-HPLC is 55% (Fig 4).

### EXAMPLE (9): DE-ACM OF THE GUANYLATED PEPTIDE AMIDE USING IODINE

Same as in Example (4), except that the Acm group protection of cysteine is removed from the guanylated peptide amide by treatment with iodine.

The peptide amide (9.18mg, estimated by Lowry's method, of Cys-Acm)is dissolved in 17.8ml of acetic acid (80%) and purged with N₂ for 15mins. 1mM solution of I₂ (in 80%acetic acid, ∼4ml)is added to the peptide solution, over a period of 1hr, till there is a persistent yellow color. The mixture is stirred for an additional 30-mins followed by neutralisation with 1N Na₂S₂O₃, till the yellow color disappeared, and lyophilized. Estimation of 'SH' is done by Ellman Test, which is negative indicating that removal of ACM has not been achieved.

### EXAMPLE (10): PURIFICATION OF THE DE-ACM PEPTIDE AMIDES

Peptide amide samples obtained in the example 8 and 9 were desalted by RP-HPLC, using the hyperprep (250 x 10mm, 12µ,C-18 column).

### EXAMPLE (11): PLATELET AGGREGATION INHIBITION ASSAY TO CHECK THE BIOACTIVITY OF FORMULA (1)

The bioactivity of peptide amide of formula (1) is checked using platelet aggregation inhibition assay using 4X Laser Aggregometer (EMA). Freshly venous blood from consented human donors are drawn and collected in citrated buffer. The platelet rich plasma (PRP) and platelet poor plasma are separated by centrifugation. Platelet count in PRP is adjusted to 2-3 x10⁸ platelets / ml. After adjusting the baseline aggregation with PPP, the PRP was treated with 10-20 mM ADP and checked the percent total aggregation. The PRP is then first incubated with varying concentrations of reference standard and synthesized peptide amide of formula (1). ADP is then added to check the inhibition of aggregation. The reproducibility of bioactivity of synthesized peptide amide of formula (1) is checked several times and compared with reference standards. Table 1 represents one of many experiments (from 12 experiments). The IC₅₀ dose for synthesized peptide (SP) was less than 140nM as compared to commercial reference standard. There is more than 50% inhibition of ADP induced platelet aggregation with SP seen in most of the samples and results are comparable with commercial reference standard.

**Table 1: Inhibition of ADP induced aggregation by synthesized peptide amide (SP) of formula (1)**

| **Donor Number** | **Percent Aggregation by ADP** | **Concentration (nM)** | **% Inhibition by** | |
|---|---|---|---|---|
| | | | **SP of formula 1** | **Reference Standard** |
| 1. | 89.4% | 70 | ND | ND |
| | | 140 | 44.6 | 31.20 |
| | | 280 | 61.40 | 52.50 |
| | | | | |
| 2. | 92.13 | 70 | 46.66 | 30.31 |
| | | 140 | 51.16 | 45.18 |
| | | 280 | 67.74 | 60.92 |
| | | | | |
| 3. | 54.14 | 70 | 57.42 | 27.41 |
| | | 140 | 60.28 | 49.20 |
| | | 280 | ND | ND |
| | | | | |
| 4. | 68.11 | 70 | ND | ND |
| | | 140 | 20.52 | 23.06 |
| | | 280 | 42.73 | 40.53 |
| | | | | |
| 5. | 66.50 | 70 | 52.63 | 45.68 |
| | | 140 | 87.21 | 57.44 |
| | | 280 | ND | ND |
| | | | | |
| 6. | 66.17 | 70 | ND | ND |
| | | 140 | 82.92 | 70.56 |
| | | 280 | ND | ND |

## Claims

1. A process for the preparation of a peptide amide N⁶-(aminoiminomethyl)-N²-(3-mercapto-1-oxopropyl-L-lysylglycyl-L-α-aspartyl-L-tryptophyl-L-prolyl-L-cysteinamide, cyclic(1→6)-disulfide of formula (1) on a solid phase, 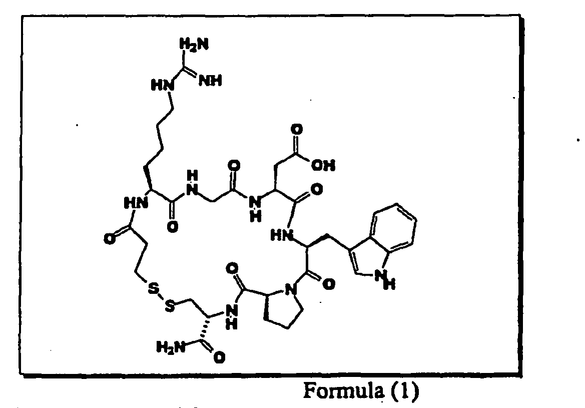 the said process comprising:
a. assembling a peptide chain comprising of six amino acids and a thioalkyl carboxylic acid in a required sequence on a solid support resin, by coupling to directly join one another by peptide bonds to obtain a peptide bound resin of formula (2) as given below:
(Acm)Mpr-Lys(Boc)-Gly-Asp(Obut)-Trp-Pro-Cys(Acm)-Resin Formula (2)
b. capping the free amino groups after each coupling of step (a) with acetic anhydride;
c. cleaving and deprotecting, all groups except Acm group, of the peptide of step (a) from the resin to obtain peptide amide of formula (3) as given below:
(Acm)Mpr-Lys-Gly-Asp-Trp-Pro-Cys(Acm)-CONH₂ Formula (3)
d. guanylating the peptide amide of formula (3) at ε-lysine-NH₂ in an organic solvent followed by precipitating with another solvent to obtain peptide amide of formula (4) as given below;
(Acm)Mpr-Homoarg-Gly-Asp-Trp-Pro-Cys(Acm)-CONH₂ Formula (4)
e. treating the peptide amide of Formula (4) with a heavy metal salt in an appropriate solvent, followed by precipitation of the heavy metal-peptide salt using an organic solvent to obtain the heavy metal-peptide salt of formula (5); 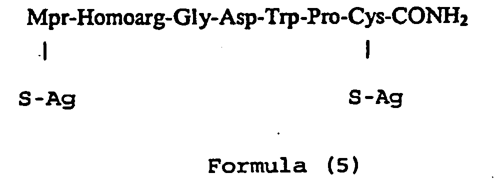
f. Oxidation and desalting of the heavy metal peptide salt of step (e) with appropriate nucleophillic reagent to obtain a peptide amide of formula (1);
g. purifying the crude peptide of step (f) by RP-HPLC.

2. A process according to claim 1, wherein the reaction of amino and carboxylic equivalent of compounds forms the said peptide bond.

3. A process according to claim 1 or 2, wherein the C-terminal of the protected first amino acid is bound to a solid phase through a linker to obtain a solid phase bound amino acid.

4. A process according to any one of the preceding claims, wherein the solid support used is an amide resin.

5. A process according to any one of the preceding claims, wherein the first protected amino acid is a thiol protected Fmoc Cysteine.

6. A process according to any one of the preceding claims, wherein the cleavage of the resin with the linker leads to the release of assembled peptide amide.

7. A process according to any one of the preceding claims, wherein the peptide amide compound is a compound joined to each of the terminal functionalities by a peptide bond and wherein each terminal functionalities is an amino or carboxylic acid group.

8. A process according to any one of the preceding claims, wherein the amino acids used are selected from the group consisting of Cys, Pro, Trp, Asp, Lys, Gly, Arg, Har, Leu and Glu.

9. A process according to any one of the preceding claims, wherein the thioalkyl carboxylic acid used is 2- thiopropionic acid.

10. A process according to any one of the preceding claims, wherein the protecting group for -NH₂ functional group of an amino acid is Fmoc or Boc.

11. A process according to any one of the preceding claims, wherein the -COOH functional group is unprotected or protected O-tBu ester.

12. A process according to any one of the preceding claims, wherein the protecting group for SH-function is Acm group.

13. A process according to any one of the preceding claims, wherein in step (c), the peptide is cleaved from solid support resin using reagents selected from the group consisting of TFA(85-98%) : TIS(0-5%) : H₂O(0-5%) : EDT(0-5%): Phenol(0-5%) and TFA(94.5-95.5%) : TIS(0-2.5%) : H₂O(0-3%) : EDT(0-2.5%).

14. A process according to any one of the preceding claims, wherein in step (d), the organic solvent used for guanylation is selected from the group consisting of DMF, ethanol and methanol.

15. A process according to any one of the preceding claims, wherein in the step (d), the precipitation of the peptide amide of formula (4) is carried out by using a solvent selected from the group consisting of acetone, acetonitrile, methanol, ethers, pentane, hexane and mixture thereof.

16. A process as claimed in claim 15, wherein the precipitation is performed using acetonitrile.

17. A process according to any one of the preceding claims, wherein in the step (g), the peptide amide of formula (1) obtained has purity of more than 99%.

18. A process according to any one of the preceding claims, wherein the preparation of the peptide amide of formula (1) by solid phase synthesis is carried out using Fmoc chemistry.

19. A process according to any one of the preceding claims, wherein in the step (d), the guanylation of peptide amide of formula (3) is performed by using DMF as the solvent.

20. A process according to any one of the preceding claims, wherein the purification of the peptide amide of Formula (4) is performed by RP-HPLC.

21. A process according to any one of the preceding claims, wherein the heavy metal salt used for the treatment of peptide amide of Formula (4) is silver trifluoromethane sulphonate in TFA.

22. A process according to any one of the preceding claims, wherein the precipitation of the heavy metal-peptide salt of Formula (5) is carried out using diisopropyl ether.

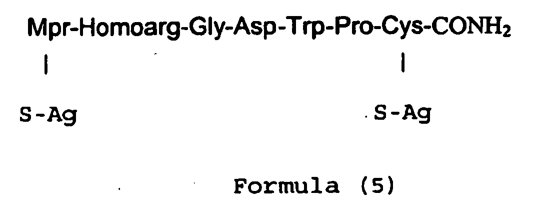

23. A process according to any one of the preceding claims, wherein in step (f), the heavy metal-peptide salt is treated with HCl and DMSO to simultaneously remove the heavy metal and to oxidize the resulting peptide to yield a crude peptide amide of Formula (1).

24. A process according to any one of the preceding claims, wherein the crude peptide amide of Formula (1) is purified by RP-HPLC.

25. A process according to any one of the preceding claims, wherein the purification of crude peptide amide of formula (1) is performed by RP-HPLC using C-4, C-8 or C-18 silica or polymer reverse phase columns using methanol and/or acetonitrile in isolation or combination with aqueous TFA(0-0.5%) as the mobile phase.

26. An intermediate peptide of formula (2)
(Acm)Mpr-Lys(Boc)-Gly-Asp(Obut)-Trp-Pro-Cys(Acm)-Resin Formula (2)

27. An intermediate peptide amide of formula (3)
(Acm)Mpr-Lys-Gly-Asp-Trp-Pro-Cys(Acm)-CONH₂ Formula (3)

28. An intermediate peptide amide of formula (4)
(Acm)Mpr-Homoarg-Gly-Asp-Trp-Pro-Cys(Acm)-CONH₂ Formula (4)

29. An intermediate peptide salt of the formula (5)

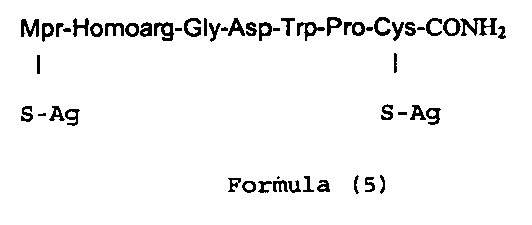

## Patentansprüche

1. Verfahren zur Herstellung eines Peptidamids in Form eines cyclischen (1→6)-Disulfids von N⁶-(Aminoiminomethyl)-N²-(3-mercapto-1-oxopropyl-L-lysylglycyl-L-α-aspartyl-L-tryptophyl-L-prolyl-L-cysteinamid der Formel (1) auf einer festen Phase 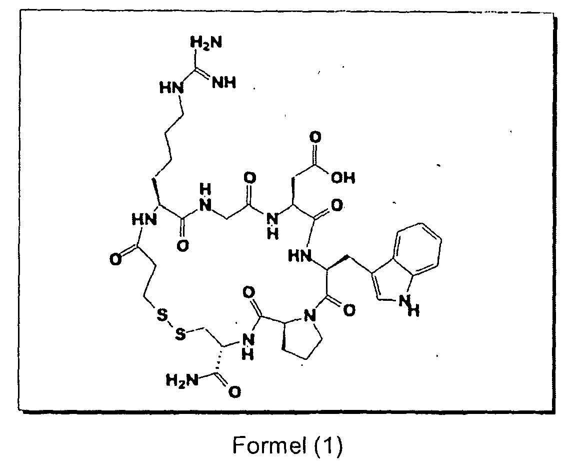 wobei das Verfahren die folgenden Stufen umfasst:
a. Zusammenbauen einer sechs Aminosäuren und eine Thioalkylcarbonsäure umfassenden Peptidkette in einer erforderlichen Sequenz auf einem festen Trägerharz durch Kuppeln zum direkten Verbinden aneinander mittels Peptidbindungen, um ein Peptid-gebundenes Harz der Formel (2) gemäß nachfolgender Formel zu erhalten:
(Acm)Mpr-Lys(Boc)-Gly-Asp(Obut)-Trp-Pro-Cys(Acm)-Harz Formel (2),
b. Überkappen der freien Aminogruppen nach jeder Kupplung der Stufe (a) mit Essigsäureanhydrid;
c. Spalten und Entschützen aller Gruppen mit Ausnahme der Acm-Gruppe, des Peptids der Stufe (a) von dem Harz, um ein Peptidamid der nachfolgenden Formel (3) zu erhalten:
(Acm)Mpr-Lys-Gly-Asp-Trp-Pro-Cys(Acm)-CONH₂ (Formel 3)
d. Guanylieren des Peptidamids der Formel (3) am ε-Lysin-NH₂ in einem organischen Lösemittel, gefolgt von einem Fällen mit einem anderen Lösemittel, um ein Peptidamid der nachfolgenden Formel (4) zu erhalten:
(Acm)Mpr-Homoarg-Gly-Asp-Trp-Pro-Cys(Acm)-CONH₂ Formel (4)
e. Behandeln des Peptidamids der Formel (4) mit einem Schwermetallsalz in einem geeigneten Lösemittel, gefolgt von einem Fällen des Schwermetall-Peptidsalzes unter Verwendung eines organischen Lösemittels, um das Schwermetall-Peptidsalz der Formel (5) zu erhalten: 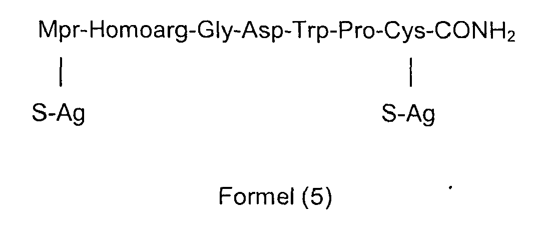
f. Oxidieren und Entsalzen des Schwermetall-Peptidsalzes der Stufe (e) mit einem geeigneten nukleophilen Reagenz, um ein Peptidamid der Formel (1) zu erhalten; und
g. Reinigen des rohen Peptids der Stufe (f) mittels RPHPLC.

2. Verfahren nach Anspruch 1, wobei die Reaktion des Amino- und Carbonsäureäquivalents der Verbindungen die Peptidbindung bildet.

3. Verfahren nach Anspruch 1 oder 2, wobei der C-Terminus der geschützten ersten Aminosäure durch einen Linker an eine feste Phase gebunden wird, um eine an eine feste Phase gebundene Aminosäure zu erhalten.

4. Verfahren nach einem der vorhergehenden Ansprüche, wobei der verwendete feste Träger ein Amidharz ist.

5. Verfahren nach einem der vorhergehenden Ansprüche, wobei die erste geschützte Aminosäure ein Thiol-geschütztes Fmoc-Cystein ist.

6. Verfahren nach einem der vorhergehenden Ansprüche, wobei die Spaltung des Harzes mit dem Linker zur Freisetzung eines zusammengebauten Peptidamids führt.

7. Verfahren nach einem der vorhergehenden Ansprüche, wobei die Peptidamidverbindung eine Verbindung ist, die durch eine Peptidbindung an jede der terminalen Funktionalitäten gebunden ist, und wobei jede terminale Funktionalität eine Amino- oder Carbonsäuregruppe ist.

8. Verfahren nach einem der vorhergehenden Ansprüche, wobei die verwendeten Aminosäuren aus der Gruppe ausgewählt sind, die aus Cys, Pro, Trp, Asp, Lys, Gly, Arg, Har, Leu und Glu besteht.

9. Verfahren nach einem der vorhergehenden Ansprüche, wobei die verwendete Thioalkylcarbonsäure 2-Thiopropionsäure ist.

10. Verfahren nach einem der vorhergehenden Ansprüche, wobei die Schutzgruppe für eine funktionale NH₂-Gruppe einer Aminosäure Fmoc oder Boc ist.

11. Verfahren nach einem der vorhergehenden Ansprüche, wobei die funktionale -COOH nicht geschützt ist oder ein geschützter O-tert-Bu-Ester ist.

12. Verfahren nach einem der vorhergehenden Ansprüche, wobei die Schutzgruppe für die SH-Funktion eine Acm-Gruppe ist.

13. Verfahren nach einem der vorhergehenden Ansprüche, wobei in Stufe (c) das Peptid von dem festen Trägerharz unter Verwendung von Reagenzien abgespalten wird, die aus der Gruppe ausgewählt sind, die aus TFA(85-98%) : TIS(0-5%) : H₂O(0-5%) : EDT(0-5%) : Phenol(0-5%) und TFA(94,5-95,5%) : TIS(0-2,5%) : H₂O(0-3%) : EDT(0-2,5%) besteht.

14. Verfahren nach einem der vorhergehenden Ansprüche, wobei in Stufe (d) das für die Guanylierung verwendete organische Lösemittel aus der Gruppe ausgewählt ist, die aus DMF, Ethanol und Methanol besteht.

15. Verfahren nach einem der vorhergehenden Ansprüche, wobei in Stufe (d) die Fällung des Peptidamids der Formel (4) unter Verwendung eines Lösemittels durchgeführt wird, das aus der Gruppe ausgewählt ist, die aus Aceton, Acetonitril, Methanol, Ethern, Pentan, Hexan und Gemischen hiervon besteht.

16. Verfahren nach Anspruch 15, wobei die Fällung unter Verwendung von Acetonitril durchgeführt wird.

17. Verfahren nach einem der vorhergehenden Ansprüche, wobei in Stufe (g) das erhaltene Peptidamid der Formel (1) eine Reinheit von mehr als 99 % aufweist.

18. Verfahren nach einem der vorhergehenden Ansprüche, wobei die Herstellung des Peptidamids der Formel (1) durch Festphasensynthese unter Verwendung einer Fmoc-Chemie durchgeführt wird.

19. Verfahren nach einem der vorhergehenden Ansprüche, wobei in Stufe (d) die Guanylierung des Peptidamids der Formel (3) unter Verwendung von DMF als Lösemittel durchgeführt wird.

20. Verfahren nach einem der vorhergehenden Ansprüche, wobei die Reinigung des Peptidamids der Formel (4) durch RP-HPLC durchgeführt wird.

21. Verfahren nach einem der vorhergehenden Ansprüche, wobei das zur Behandlung des Peptidamids der Formel (4) verwendete Schwermetallsalz ein Silbertrifluormethansulfonat in TFA ist.

22. Verfahren nach einem der vorhergehenden Ansprüche, wobei die Fällung des Schwermetall-Peptidsalzes der Formel (5) unter Verwendung von Diisopropylether durchgeführt wird.

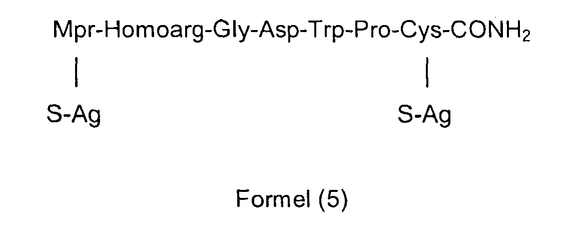

23. Verfahren nach einem der vorhergehenden Ansprüche, wobei in Stufe (f) das Schwermetall-Peptidsalz mit HCl und DMSO behandelt wird, um gleichzeitig das Schwermetall zu entfernen und das erhaltenen Peptid unter Bildung eines rohen Peptidamids der Formel (1) zu oxidieren.

24. Verfahren nach einem der vorhergehenden Ansprüche, wobei das rohe Peptidamid der Formel (1) durch RP-HPLC gereinigt wird.

25. Verfahren nach einem der vorhergehenden Ansprüche, wobei die Reinigung des rohen Peptidamids der Formel (1) durch RP-HPLC unter Verwendung von C-4-, C-8- oder C-18-Silica- oder -Polymerumkehrphasensäulen unter Verwendung von Methanol und/oder Acetonitril in Isolation oder Kombination mit wässriger TFA(0-0,5%) als mobiler Phase durchgeführt wird.

26. Peptidzwischenprodukt der Formel (2)
(Acm)Mpr-Lys(Boc)-Gly-Asp(Obut)-Trp-Pro-Cys(Acm)-Harz Formel (2),

27. Peptidamidzwischenprodukt der Formel (3)
(Acm)Mpr-Lys-Gly-Asp-Trp-Pro-Cys(Acm)-CONH₂ (Formel 3)

28. Peptidamidzwischenprodukt der Formel (4)
(Acm)Mpr-Homoarg-Gly-Asp-Trp-Pro-Cys(Acm)-CONH₂ Formel (4)

29. Peptidsalzzwischenprodukt der Formel (5)

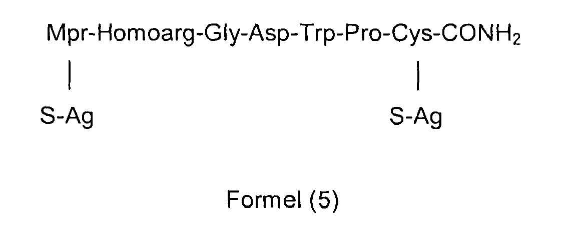

## Revendications

1. Procédé pour la préparation d'un amide peptide N⁶-(aminoiminométhyl)-N²-(3-mercapto-1-oxopropyl-L-lysylglycyl-L-α-aspartyl-L-tryptophyl-L-prolyl-L-cystéinamide, disulfure cyclique (1→6) de formule (1) sur une phase solide, 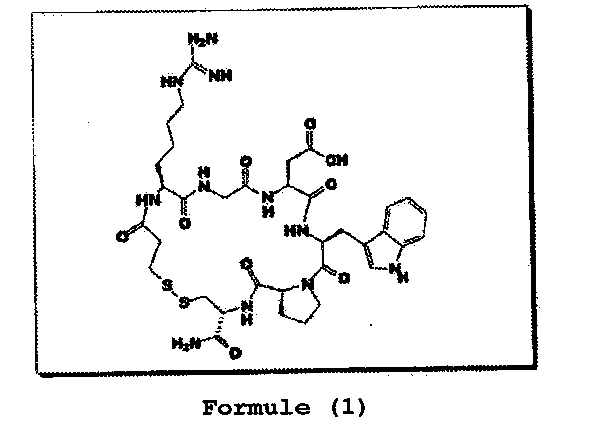 ledit procédé comprenant les étapes de :
a. assembler une chaîne peptide comprenant six acides aminés et un acide carboxylique thioalkylé en une séquence requise sur une résine à support solide, par couplage afin de les unir directement les uns aux autres par des liaisons peptidiques pour obtenir une résine liée au peptide de formule (2) telle qu'indiquée ci-dessous :
(Acm)Mpr-Lys(Boc)-Gly-Asp(Obut)-Trp-Pro-Cys(Acm)-résine Formule (2)
b. coiffer les groupes amino libres après chaque couplage de l'étape (a) avec de l'anhydride acétique ;
c. cliver et déprotéger tous les groupes, à l'exception du groupe Acm, du peptide de l'étape (a) de la résine pour obtenir un amide peptide de formule (3) telle qu'indiquée ci dessous :
(Acm)Mpr-Lys-Gly-Asp-Trp-Pro-Cys(Acm)-CONH₂ Formule (3)
d. guanyler l'amide peptide de formule (3) sur ε-lysine-NH₂ dans un solvant organique, puis réaliser une précipitation avec un autre solvant pour obtenir un amide peptide de formule (4) telle qu'indiquée ci-dessous :
(Acm)Mpr-Homoarg-Gly-Asp-Trp-Pro-Cys(Acm)-CONH₂ Formule (4)
e. traiter l'amide peptide de formule (4) avec un sel de métal lourd dans un solvant approprié, puis réaliser une précipitation du sel métal lourd-peptide en utilisant un solvant organique pour obtenir le sel de métal lourd-peptide de formule (5) : 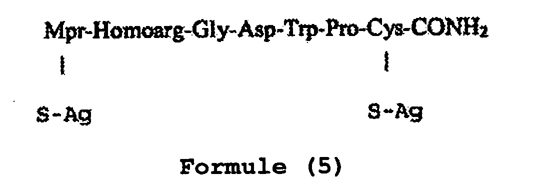
f. oxyder et désaler le sel de métal lourd-peptide de l'étape (e) avec un réactif nucléophile approprié pour obtenir un amide peptide de formule (1) ;
g. purifier le peptide brut de l'étape (f) par RP-HPLC.

2. Procédé selon la revendication 1, dans lequel la réaction des équivalents aminés et carboxylique des composés forme ladite liaison peptidique.

3. Procédé selon la revendication 1 ou 2, dans lequel le C-terminal du premier acide aminé protégé est lié à une phase solide par un élément de liaison afin pour obtenir un acide aminé lié à la phase solide.

4. Procédé selon l'une quelconque des revendications précédentes, dans lequel le support solide utilisé est une résine amidique.

5. Procédé selon l'une quelconque des revendications précédentes, dans lequel le premier acide aminé protégé est une cystéine Fmoc thiol protégée.

6. Procédé selon l'une quelconque des revendications précédentes, dans lequel le clivage de la résine munie de l'élément de liaison conduit à la libération d'un amide peptide assemblé.

7. Procédé selon l'une quelconque des revendications précédentes, dans lequel le composé amide peptide est un composé lié à chacune des fonctionnalités terminales par une liaison peptidique et dans lequel chaque fonctionnalité terminale est un groupe amino ou acide carboxylique.

8. Procédé selon l'une quelconque des revendications précédentes, dans lequel les acides aminés utilisés sont choisis parmi le groupe constitué de Cys, Pro, Trp, Asp, Lys, Gly, Arg, Har, Leu et Glu.

9. Procédé selon l'une quelconque des revendications précédentes, dans lequel l'acide carboxylique thioalkylé utilisé est l'acide 2-thiopropionique.

10. Procédé selon l'une quelconque des revendications précédentes, dans lequel le groupe protecteur pour le groupe fonctionnel -NH₂ d'un acide aminé est Fmoc ou Boc.

11. Procédé selon l'une quelconque des revendications précédentes, dans lequel le groupe fonctionnel -COOH est non protégé ou un ester O-tBu-protégé.

12. Procédé selon l'une quelconque des revendications précédentes, dans lequel le groupe protecteur pour la fonction SH est le groupe Acm.

13. Procédé selon l'une quelconque des revendications précédentes, dans lequel à l'étape (c), le peptide est clivé de la résine à support solide en utilisant des réactifs choisis parmi le groupe constitué de TFA (85 à 98 %) : TIS (0 à 5 %) : H₂O (0 à 5 %) : EDT (0 à 5 %) : phénol (0 à 5 %) et TFA (94,5 à 95,5 %) : TIS (0 à 2,5 %) : H₂O (0 à 3 %) : EDT (0 à 2,5 %).

14. Procédé selon l'une quelconque des revendications précédentes, dans lequel à l'étape (d), le solvant organique utilisé pour la guanylation est choisi parmi le groupe constitué de DMF, d'éthanol et de méthanol.

15. Procédé selon l'une quelconque des revendications précédentes, dans lequel à l'étape (d), la précipitation de l'amide peptide de formule (4) est réalisée en utilisant un solvant choisi parmi le groupe constitué d'acétone, d'acétonitrile, de méthanol, d'éthers, de pentane, d'hexane et de mélange de ceux-ci.

16. Procédé selon la revendication 15, dans lequel la précipitation est réalisée en utilisant l'acétonitrile.

17. Procédé selon l'une quelconque des revendications précédentes, dans lequel à l'étape (g), l'amide peptide de formule (1) obtenu a une pureté supérieure à 99 %.

18. Procédé selon l'une quelconque des revendications précédentes, dans lequel la préparation de l'amide peptide de formule (1) par synthèse en phase solide est exécutée en utilisant une chimie Fmoc.

19. Procédé selon l'une quelconque des revendications précédentes, dans lequel à l'étape (d), la guanylation de l'amide peptide de formule (3) est réalisée en utilisant du DMF comme solvant.

20. Procédé selon l'une quelconque des revendications précédentes, dans lequel la purification de l'amide peptide de formule (4) est réalisée par RP-HPLC.

21. Procédé selon l'une quelconque des revendications précédentes, dans lequel le sel de métal lourd utilisé pour le traitement de l'amide peptide de formule (4) est du trifluorométhane sulfonate d'argent dans du TFA.

22. Procédé selon l'une quelconque des revendications précédentes, dans lequel la précipitation du sel de métal lourd-peptide de formule (5) est effectuée en utilisant de l'éther diisopropylique.

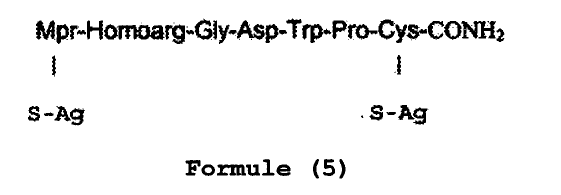

23. Procédé selon l'une quelconque des revendications précédentes, dans lequel à l'étape (f), le sel de métal lourd-peptide est traité à l'aide de HCl et DMSO pour simultanément éliminer le métal lourd et oxyder le peptide résultant pour produire un amide peptide brut de formule (1).

24. Procédé selon l'une quelconque des revendications précédentes, dans lequel l'amide peptide brut de formule (1) est purifié par RP-HPLC.

25. Procédé selon l'une quelconque des revendications précédentes, dans lequel la purification de l'amide peptide brut de formule (1) est réalisée par RP-HPLC en utilisant des colonnes à phase inversée de polymère ou de silice C-4, C-8 ou C-18 en utilisant du méthanol et/ou de l'acétonitrile isolément ou en combinaison avec du TFA aqueux (0 à 0,5 %) en tant que phase mobile.

26. Peptide intermédiaire de formule (2)
(Acm)Mpr-Lys(Boc)-Gly-Asp(Obut)-Trp-Pro-Cys(Acm)-résine Formule (2)

27. Amide peptide intermédiaire de formule (3)
(Acm)Mpr-Lys-Gly-Asp-Trp-Pro-Cys(Acm)-CONH₂ Formule (3)

28. Amide peptide intermédiaire de formule (4)
(Acm)Mpr-Homoarg-Gly-Asp-Trp-Pro-Cys(Acm)-CONH₂ Formule (4)

29. Sel de peptide intermédiaire de formule (5)
